Europäisches Patentamt

European Patent Office

Office européen des brevets

(19)

(11) Numéro de publication : **0 028 546**
**B1**

(12) **FASCICULE DE BREVET EUROPÉEN**

(45) Date de publication du fascicule du brevet :
25.07.84

(51) Int. Cl.³ : **A 61 F   1/03**

(21) Numéro de dépôt : **80401377.9**

(22) Date de dépôt : **30.09.80**

(54) **Prothèse de hanche.**

(30) Priorité : **03.10.79 FR 7924589**

(43) Date de publication de la demande :
**13.05.81 Bulletin 81/19**

(45) Mention de la délivrance du brevet :
**25.07.84 Bulletin 84/30**

(84) Etats contractants désignés :
**AT BE CH DE GB IT LI LU NL SE**

(56) Documents cités :
**EP-A- 0 013 863**
**DE-A- 2 535 649**
**DE-A- 2 559 402**
**DE-A- 2 807 289**
**DE-B- 1 164 019**
**DE-C-   876 739**
**DE-C-   976 768**
**FR-A- 2 300 542**
**FR-A- 2 416 004**
**US-A- 4 077 070**

(73) Titulaire : **Teinturier, Pierre Champeaux**
**F-63110 Beaumont (FR)**

(72) Inventeur : **Teinturier, Pierre Champeaux**
**F-63110 Beaumont (FR)**

(74) Mandataire : **Pinguet, André**
**CAPRI 28 bis, avenue Mozart**
**F-75016 Paris (FR)**

EP 0 028 546 B1

Jouve, 18, rue St-Denis, 75001 Paris, France

## Description

La présente invention a pour objet une prothèse de hanche constituant une articulation de la tête du fémur sur le bassin. La prothèse comporte un élément cotyloïdien prothétique et un élément fémoral ; l'élément cotyloïdien est prévu pour remplacer la cavité cotyloïde dégradée du bassin. L'élément fémoral permet de reconstituer une tête fémorale sphérique adaptée à l'élément cotyloïdien.

Jusqu'ici les prothèses de hanche comportent un élément cotyloïdien mis en place dans la cavité cotyloïde du bassin ; le scellement est assuré soit par du ciment de type métacrylate de méthyle, soit par l'irrégularité de la surface en contact avec l'os, irrégularité réalisée par de multiples procédés (chouppage, billes, stries, etc...). On connaît en particulier, par le document FR 2 416 004, une prothèse comportant une cupule métallique sphérique formée avec un certain nombre de fentes radiales et dont l'extérieur est garni d'un revêtement comportant des aspérités. Pour la mise en place, la cupule métallique est saisie à l'aide d'un outil préhenseur qui permet en raison de l'élasticité conférée à la cupule par la présence des fentes radiales d'obtenir une diminution du diamètre externe et de permettre ainsi son introduction dans la cavité cotyloïdienne préalablement préparée. Après relâchement, la cupule se fixe grâce à son élasticité, par pénétration des aspérités dans l'os sous-chondral. Il faut ensuite introduire à l'intérieur une hémisphère en matière plastique.

L'inconvénient de ces procédés tient au fait que les déformations de l'os au cours de la marche normale sont ignorées. Ni le métacrylate de méthyle ni le métal n'ont le même module d'Young que l'os et les formes des prothèses ne permettent pas les mouvements de déformation pouvant s'adapter aux déformations de l'os.

Ces inconvénients sont supprimés, conformément à la présente invention qui propose une prothèse de la hanche comportant un élément cotyloïdien constitué en un matériau déformable élastiquement, cet élément ayant au moins une face plane dans laquelle est ménagée une cavité sphérique, des sections droites circulaires et une échancrure latérale, qui par rapprochement de ses bords par une contrainte appropriée, permet de réduire l'encombrement de l'élément, afin de pouvoir l'engager dans une cavité de forme complémentaire et de dimensions inférieures creusée dans l'os du bassin du patient auquel la prothèse est destinée, le calage de l'élément dans ladite cavité étant assuré élastiquement par le relâchement de la contrainte ayant servi à rapprocher les bords de l'échancrure, remarquable notamment en ce que l'élément est constitué par une portion de cylindre et en ce que l'échancrure est ménagée longitudinalement le long de génératrices et d'un bout à l'autre de ladite portion de cylindre de manière à conférer à l'élément une forme générale en fer à cheval.

Le même problème se pose au niveau de la prothèse fémorale où la fixation de la prothèse est assurée dans le canal médullaire de l'os soit par, comme au niveau du bassin, du métacrylate de méthyle qui compense le défaut d'ajustage de la queue de la prothèse fémorale au diamètre de la cavité médullaire, soit par une prothèse métallique de diamètre plus important et comportant comme au niveau du bassin des irrégularités destinées à une fixation correcte de l'os sur ces irrégularités.

La prothèse fémorale entrant dans le cadre de cette invention est destinée à se fixer non pas dans la cavité médullaire mais dans le tissu spongieux de la tête et du col fémoral, en utilisant un matériau dont le module de Jung se rapproche du tissu spongieux (polyéthylène haute densité).

Conformément à la présente invention, la prothèse peut comporter en outre une tête fémorale prothétique comportant une tête avec une tige ou ancre supérieure, en matière flexible, la tige étant orientée par rapport à la tête pour suivre le bord supérieur du col fémoral, la partie distale de cette ancre supérieure étant fendue ou bifide pour conférer une élasticité de maintien en place par rapprochement des deux éléments de la bifidité, la tête étant percée d'un orifice légèrement conique pour le passage d'une ancre inférieure orientée sensiblement dans la longueur du fémur vers sa corticale interne.

Sur les deux prothèses de petites ailettes de polyéthylène vont après introduction dans le tissu spongieux, grâce à leur élasticité, se ficher dans le tissu spongieux ; une prolifération osseuse va s'effectuer autour de ces languettes polyéthylène et maintenir en place à longue échéance les éléments prothétiques.

Au niveau du bassin, il existe de plus l'utilisation du phénomène de clips qui applique de manière dynamique l'élément cotyloïdien à la cavité destinée à le recevoir.

La description qui va suivre, en regard des dessins annexés, donnée à titre d'exemple non limitatif, fera bien comprendre comment l'invention peut être réalisée.

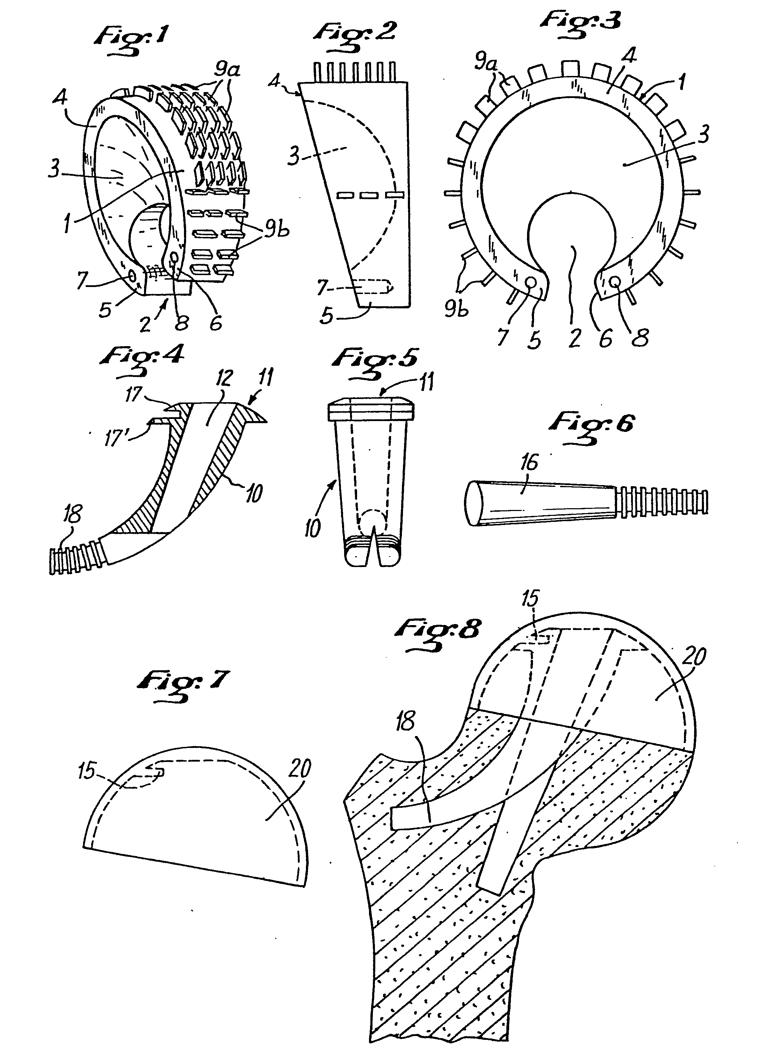

La figure 1 est une vue en perspective d'un élément cotyloïdien selon l'invention ;

La figure 2 est une vue en élévation de profil de l'élément de la figure 1 ;

La figure 3 est une vue en élévation de face du même élément ;

La figure 4 est une vue en coupe d'une ancre supérieure d'une prothèse fémorale ;

La figure 5 est une vue en élévation à 90° de la vue précédente ;

La figure 6 est une vue en élévation d'une ancre inférieure de la prothèse fémorale ;

La figure 7 est une vue en élévation d'une cupule pour cette prothèse et

La figure 8 est une vue d'ensemble, avec coupe partielle de la prothèse fémorale.

Le dispositif décrit ci-dessous est constitué par des éléments de prothèse de hanche dont l'implantation ne nécessite aucun ciment de type métacrylate de méthyle ou matériaux analogues. Il comporte deux éléments :

— un élément cotyloïdien
— un élément fémoral.

L'élément cotyloïdien

Le cotyle prothétique en polyéthylène haute densité ou autre matériau de synthèse (polyéthylène chargé, par exemple en graphite, fibre de carbone, bisulfure de molybdène, etc.) est usiné de manière telle que sa mise en place assure une fixation osseuse directe sans intermédiaire de ciment.

Le cotyle prothétique 1 est constitué par une pièce de forme cylindrique, délimitée axialement par deux plans, dont un, le plan axial, est normal à l'axe de révolution, et l'autre, le plan latéral, peut faire avec l'axe un angle d'environ 15°. Observé axialement (figure 3), le cotyle a un profil extérieur d'ensemble circulaire, et comporte une échancrure 2, le cotyle ayant ainsi sous cet angle un aspect du type fer à cheval.

Dans la face latérale est usinée (ou formée autrement) une cavité sphérique 3, servant de surface d'assise d'une articulation sphérique. Une partie plane 4 peut être laissée autour de la cavité, et dans les bords 5, 6 ou cornes de l'échancrure sont prévus deux trous 7, 8 pour y placer un outil servant à resserrer le cotyle, à la manière d'un clip. Afin de placer le cotyle dans le bassin du sujet, une cavité cylindrique de dimensions appropriées est creusée dans l'os du bassin, à l'emplacement de l'articulation détériorée à renouveler. Les dimensions du cylindre osseux correspondent à celles du cotyle de façon que celui-ci soit maintenu élastiquement après y avoir été enfoncé alors qu'il est restreint.

Pour favoriser la fixation du cotyle, outre l'extension donnée par le phénomène de clips et qui maintient la périphérie du cotyle contre l'os, la garniture des languettes de polyéthylène flexibles assure à longue échéance une fixation osseuse sur ces languettes, étant donné que le matériau utilisé (polyéthylène haute densité) a un module de Young proche de celui de l'os cotyloïdien, les modifications de forme du cotyle qui surviennent à chaque pas seront transmises à la prothèse de manière harmonieuse à l'os spongieux entourant la prothèse. Ces languettes 9, de 0,2 à 2 mm d'épaisseur, de 2 à 3 mm de hauteur, sont placées en rangées, séparées les unes des autres soit par quelques millimètres soit par un simple trait.

La disposition dans l'espace de ces languettes permet de les diviser en deux groupes :

— un groupe supérieur
— un groupe inférieur.

Le groupe supérieur occupe la moitié ou le tiers supérieur de la périphérie du cotyle. Il est fait de languettes 9a disposées dans un plan radial (soit parallèle au fond de la pièce prothétique). Le blocage intra-osseux de ces languettes est destiné à stabiliser le cotyle prothétique dans le plan frontal.

Le groupe inférieur occupe le tiers ou le quart antérieur et le tiers ou le quart postérieur de la périphérie du cotyle. Il est fait de languettes 9b disposées orthogonalement aux languettes du groupe supérieur, soit, dans des plans passant par l'axe de la prothèse cotyloïdienne.

Cette disposition des languettes est prévue en fonction du mode de mise en place du cotyle dans le cylindre osseux. On introduit d'abord la partie inférieure, quand les deux bords 5 et 6 sont rapprochés au moyen d'un outil approprié, tel qu'une pince à clip, enfoncé dans les trous 7 et 8. C'est pourquoi les languettes inférieures sont orientées dans des plans passant par l'axe du cylindre formant le cotyle. Une fois la partie inférieure engagée à fond dans le cylindre osseux, on bascule la partie supérieure par pivotement autour du point d'appui de la partie inférieure. Les languettes supérieures peuvent ainsi fléchir en passant contre la partie cylindrique de la cavité osseuse.

Afin de permettre au cotyle prothétique de suivre les mouvements qui existent au niveau des cornes antérieure et postérieure du cotyle du bassin, la partie haute de la pièce cotyloïdienne pourra recevoir des modifications de forme sous forme d'échancrure ou modifications d'épaisseur, destinées à amincir le fond ou toit dudit cotyle ; l'importance, la forme et le nombre de ces échancrures est variable en fonction du matériau utilisé, de son module de Young, du diamètre du cotyle, de l'épaisseur du toit du cotyle.

Après mise en place de la prothèse cotyloïdienne, les languettes reprennent leur position initiale par élasticité ; l'os se reformant autour de ces languettes assure une bonne liaison entre l'os et l'élément prothétique.

Le blocage de la prothèse est donc assuré :

1. dans le plan frontal, par les languettes supérieures 9a ;
2. dans le plan sagittal, par les languettes 9b des deux groupes inférieurs ;
3. dans le plan horizontal, par l'usinage en segment de cylindre du cotyle prothétique, s'engageant à force dans le segment du cylindre osseux, creusé dans le bassin.

La mise en place du cotyle prothétique utilise le procédé du clip qui, en rapprochant les cornes, permet d'engager dans le cylindre osseux, d'abord la partie inférieure de la prothèse rétrécie par le rapprochement des cornes, puis secondairement la partie supérieure avec les languettes supérieures. Après pénétration de l'élément prothétique dans le cylindre osseux, la pince à clip est ouverte, permettant, grâce à l'élasticité du matériau, l'application en force de la périphérie du cotyle sur l'os.

Le diamètre du segment de sphère, destiné à recevoir la tête fémorale prothétique est fonction du type de la prothèse fémorale utilisée qui peut être :

soit une prothèse à tige médullaire fixée ou non avec du ciment ; le diamètre d'usinage du cotyle correspondra alors au type de prothèse fémorale utilisée,

soit la prothèse fémorale ci-dessous décrite, dite cupule ancrée.

La cupule ancrée comporte trois pièces distinctes :

1)) l'ancre supérieure 10 (Fig. 4 et 5)
2) l'ancre inférieure 16 (Fig. 6)
3) la cupule 20 (Fig. 7).

1) L'ancre supérieure en polyéthylène haute densité comporte en une seule pièce 10 une plate-forme d'appui 11 sur la tête fémorale. Cette plate-forme de forme ovoïde correspond au segment de tête fémorale resséqué à la partie supérieure de la sphère fémorale. La partie supérieure du fémur est représentée hachurée sur la figure 8. Sur cette plate-forme se fixera mécaniquement la cupule par un système d'usinage soit par petites chevilles réalisant un emmanchement à frottement dur, soit par une languette intérieure 15 engagée entre deux collets 17, 17' de la tête d'ancre supérieure, soit par la réalisation à la face osseuse de la cupule d'une forme en creux adaptée à la plate-forme et réalisée en microfusion ou en usinage.

Cette plate-forme est percée d'un orifice 12 par lequel passera l'ancre inférieure 16 (Fig. 6).

Cette plate-forme se prolonge par l'ancre supérieure 18 qui va suivre le bord supérieur du col fémoral (Fig. 8). Il s'agit d'une tige de polyéthylène de diamètre décroissant, pourvue sur sa périphérie de languettes de polyéthylène disposées radialement. La partie distale de cette ancre supérieure est bifide, usinée avec un écartement des deux éléments de cette bifidité. Au niveau de cette bifidité les languettes de polyéthylène sont disposées sur une demi-circonférence, de manière telle que le rapprochement des deux hémi-tiges de la bifidité réalise une disposition circulaire complète des languettes.

La mise en place de cette ancre supérieure nécessite :

— un fantôme de résection de la tête fémorale,
— un perforateur,
— la bifidité, lors de la mise en place de l'ancre supérieure, est supprimée par rapprochement par des ligatures au fil résorbable des deux hémi-tiges constituant la bifidité distale. La résorption des fils permettra par l'élasticité du matériau la reconstitution de la bifidité initiale et la fixation en force dans le plan horizontal de la partie distale de cette ancre supérieure.

2) L'ancre inférieure est une tige de polyéthylène 16 garnie également de languettes disposées comme précédemment, sur des circonférences successives de cette tige de polyéthylène et espacées les unes des autres de quelques millimètres. D'un diamètre dégressif de haut en bas, ces deux tiges traversent l'orifice de la plate-forme de l'ancre supérieure et vont s'appuyer sur la corticale interne et le spongieux voisin (Fig. 8). Les deux ancres travaillent donc en traction pour l'ancre supérieure, en compression pour l'ancre inférieure.

3) La cupule 20 est métallique ou en matériau de synthèse compatible avec le cotyle prothétique. Elle représente un segment de sphère de deux tiers environ. L'épaisseur diminue de la partie haute à la partie basse.

A la partie haute, un usinage en creux lui permet de s'adapter à la forme de la plate-forme ; un ergot périphérique accroche le bord externe de la plate-forme et empêche les mouvements de bascule en varus de la cupule. Les bords de la plate-forme stabilisent cette cupule dans le plan sagittal. Le diamètre extérieur de la cupule correspond au diamètre intérieur de la prothèse cotyloïdienne choisie ou du cotyle, si aucune prothèse cotyloïdienne n'est utilisée. La taille des ancrages correspond à la taille de l'épiphyse fémorale considérée ; plusieurs tailles sont donc nécessaires en fonction de la morphologie du sujet.

La face de cette cupule en contact avec l'os sera réalisée de manière irrégulière afin de favoriser par la prolifération du tissu osseux, une stabilisation complémentaire.

La mise en place de cette prothèse est assurée de la manière suivante :

a) repérage de l'axe du col fémoral ;
b) sphéricisation de la tête fémorale par une fraise creuse de diamètre adapté ;
c) mise en place d'un fantôme permettant la coupe du pôle supérieur de la tête fémorale.

A l'aide de ce fantôme, le trajet de l'ancrage supérieur est réalisé, après mise en place de l'ancrage supérieur, l'ancrage inférieur est positionné à travers la plate-forme.

Enfin, la cupule est arrimée sur la plate-forme et le reste de la tête fémorale.

**Revendications**

1. Prothèse de la hanche comportant un élément cotyloïdien (1) constitué en un matériau déformable élastiquement, cet élément ayant au moins une face plane (4) dans laquelle est ménagée une cavité sphérique (3), des sections droites circulaires et une échancrure latérale (2), qui, par rapprochement de ses bords (5, 6) par une contrainte appropriée, permet de réduire l'encombrement de l'élément (1), afin de pouvoir l'engager dans une cavité de forme complémentaire et de dimensions inférieures creusée dans l'os du bassin du patient auquel la prothèse est destinée, le calage de l'élément (1) dans ladite cavité étant assuré élastiquement par le relâchement de la contrainte ayant servi à rapprocher les bords (5, 6) de l'échancrure (2) caractérisée en ce que l'élément (1) est constitué par une portion de cylindre et en ce que l'échancrure (2) est ménagée longitudinalement le long de génératrices et d'un bout à l'autre de ladite portion de cylindre de manière à conférer à l'élément une forme générale en fer à cheval.

2. Prothèse selon la revendication 1, caractérisée en ce que la portion de cylindre est délimitée par un plan normal à l'axe de la portion de cylindre sur le côté prévu pour être en contact avec l'os et par un autre plan oblique par rapport audit axe, la partie mince de l'élément se situant du côté de l'échancrure.

3. Prothèse selon une des revendications 1 et 2, caractérisée en ce qu'un trou (7, 8) est prévu dans l'élément de chaque côté de l'échancrure, pour permettre d'y engager un outil servant à rapprocher les bords de l'échancrure.

4. Prothèse selon une des revendications précédentes, dans laquelle le pourtour de l'élément est garni de languettes flexibles (9) faisant saillie radialement autour de l'élément, caractérisée en ce que les languettes (9a) disposées sur la partie de la portion de cylindre opposée à l'échancrure sont orientées dans des plans perpendiculaires à l'axe de la portion de cylindre et les languettes (9b) voisines de l'échancrure sont disposées dans des plans passant par l'axe de ladite portion de cylindre.

5. Prothèse selon une des revendications précédentes, caractérisée en ce qu'elle comporte en outre une tête fémorale prothétique (20) comportant une tête avec une tige (10) ou ancre supérieure, en matière flexible, la tige étant orientée par rapport à la tête pour suivre le bord supérieur du col fémoral, la partie distale de cette ancre supérieure étant fendue ou bifide pour conférer une élasticité de maintien en place par rapprochement des deux éléments de la bifidité, la tête étant percée d'un orifice légèrement conique pour le passage d'une ancre inférieure (16) orientée sensiblement dans la longueur du fémur vers sa corticale interne.

6. Prothèse selon la revendication 5, caractérisée en ce que l'élément cotyloïdien est en polyéthylène haute densité et en ce que la tête fémorale est recouverte d'une cupule (20) soit en matériau métallique soit en matériau de synthèse à condition que ce matériau de synthèse soit compatible sur le plan du frottement avec le polyéthylène à haute densité de la cavité cotyloïdienne.

7. Prothèse selon une des revendications précédentes, caractérisée en ce que la partie haute de l'élément cotyloïdien (1) reçoit des modifications de forme, sous forme d'échancrures ou de modifications d'épaisseur, destinées à amincir le fond ou toit dudit élément pour permettre aux cornes de l'élément de suivre le mouvement physiologique des cornes antérieure et postérieure correspondantes du bassin.

8. Prothèse selon une des revendications précédentes, caractérisée en ce que l'élément cotyloïdien est constitué d'un matériau dont le module de Young est proche de celui de l'os.

## Claims

1. Hip prosthesis comprising a cotyloidal element (1) made of an elastically deformable material, said element having at least a plane face (4) having formed therein a spherical cavity (3), circular cross-sections and a lateral identation (2), which by drawing together the edges (5, 6) by means of a proper force, allows to reduce its overall dimensions, for permitting it to be engaged into a cavity of complementary shape and smaller dimensions, bored in the pelvis bone of a patient to whom said prosthesis is intended, the clamping of element (1) in said cavity being provided elastically by release of the force which was applied to draw together the edges (5, 6) of the indentation (2), characterized in that element (1) has the form of a cylinder portion and in that the indentation (2) is formed longitudinally along generatrices from one end to the other end of said cylinder portion so as to give to the element a general horse shoe shape.

2. Prosthesis according to claim 1, characterized in that the cylinder portion is delimited by a plane perpendicular to the axis of the cylinder portion, at the end designed for being in contact with the bone, and by another plane oblique with regard to said cylinder portion axis, the thinner portion of said element being located on the indentation side.

3. Prosthesis according to one of claims 1 and 2, characterized in that a hole (7, 8) is provided in the element on each side of the indentation, for allowing to pass therein a tool for drawing together the edges of the indentation.

4. Prosthesis according to one of the preceding claims, in which the outer cylindrical surface of the element is furnished with radially outwardly projecting flexible tongues (9) characterized in that the tongues (9a), disposed on the cylinder portion surface opposite to the indentation (2), are positioned in planes perpendicular to the axis of the cylinder portion and the tongues (9b) disposed near the indentation are positioned in planes passing through the longitudinal axis of the cylindrical portion.

5. Prosthesis according to one of the preceding claims, characterized in that it further includes a prosthetic femoral head (20), comprising a head with a shank (10) or upper anchor, made of an elastically deformable material, said shank being directed to be placed along the upper edge of the femoral collar, the distal portion of said upper anchor being split or bifid for providing resilience for fixing the position by drawing together the two parts of the bifidity, said head being bored with a passage of slight conical form for receiving a lower anchor, substantially directed along the femur length towards its inner cortical.

6. Prosthesis according to claim 5, characterized in that said cotyloidal element is made of high density polyethylene and that the femoral head is lined with a cup (20), either in metallic material or in synthetic material, provided said synthetic material is frictionnally compatible with high density polyethylene of the cotyloidal cavity (3).

7. Prosthesis according to one of the preceding claims, characterized in that the upper part of cotyloidal element (1) has its form altered, by indentations of thickness alterations in view of making the bottom, or roof, of said element thinner for allowing the horns of said element to adapt themselves to physiological motion of corresponding anterior and posterior horns of pelvis.

8. Prosthesis according to one of the preceding claims, characterized in that said cotyloidal element is made of a material, the Young modulus of which is near that of the bone.

**Ansprüche**

1. Hüftegelenkprothese mit einem gelenkpfannenartigen Element (1), das aus einem elastisch verformbaren Material besteht und wenigstens eine Planfläche (4) aufweist, in welcher eine Kugelhöhlung (3), gerade Kreisabschnitte und eine seitliche Ausbuchtung (2) eingearbeitet sind, die durch Annäherung ihrer Ränder (5, 6) unter einer geeigneten Beanspruchung den Platzbedarf des Elements (1) zu mindern vermag, damit es in eine Höhlung mit ergänzender Form und kleineren Abmessungen eingeführt werden kann, die sich in der Hüftgelenkpfanne des Patienten befindet, wobei die Verankerung des Elements (1) in der genannten Höhlung federnd durch das Nachlassen der Beanspruchung gewährleistet wird, unter welcher die Ränder (5, 6) der Ausbuchtung (2) einander angenähert wurden, dadurch gekennzeichnet, dass das Element (1) aus einem Zylinderabschnitt besteht und die Ausbuchtung (2) der Länge nach längs der Mantelfläche und durchgehend am genannten Zylinderabschnitt vorgesehen ist, damit das Element allgemein ein Hufeisenprofil hat.

2. Prothese nach Anspruch 1, dadurch gekennzeichnet, das der Zylinderabschnitt durch eine Ebene quer zur Achse des Zylinderabschnitts, an der für die Knochenberührung vorgesehenen Seite, sowie durch eine andere Ebene schräg zur genannten Achse abgegrenzt ist, wobei der dünne Teil des Elements sich auf der Seite der Ausbuchtung befindet.

3. Prothese nach einem der Ansprüche 1 und 2, dadurch gekennzeichnet, dass ein Loch (7, 8) im Element auf jeder Seite der Ausbuchtung zur Aufnahme eines Werkzeugs vorgesehen ist, das die Ränder der Ausbuchtung einander annähert.

4. Prothese nach einem der vorherigen Ansprüche, in der der Umfang des Elements mit federnden Zungen (9) belegt is die radial um das Element herum vorspringen, dadurch gekennzeichnet, dass die Zungen (9a), die sich am Teil des Zylinderabschnittes befinden, der der Ausbuchtung entgegengesezt ist, in Ebenen quer zur Achse des Zylinderabschnittes ausgerichtet sind und die Zungen (9b) in der Nähe der Ausbuchtung in Ebenen liegen, die die Achse des genannten Zylinderabschnitts beinhalten.

5. Prothese nach einem der vorhergehenden Ansprüche, dadurch gekennzeichnet, dass sie ausserdem einen prothetischen Kopf (20) des Oberschenkelknochens hat, der einen Kopf mit einem oberen Stiel (10) oder Anker aus federndem Material aufweist, wobei der Stiel zum Kopf so ausgerichtet ist, dass er dem oberen Rand des Oberschenkelhases folgen kann, während der distale Teil dieses oberen Ankers geschlizt oder zweispaltig ist, was ein federndes Festhalten durch das Annähern beider Elemente der Zweispaltigkeit gewährleistet, wobei der Kopf zur Aufnahme eines unteren Ankers (16), der ungefähr nach der Länge des Oberschenkelknochens zu seiner inneren Kortikalen hin ausgerichtet ist, mit einer leicht kegeligen Offnung versehen ist.

6. Prothese nach Anspruch 5, dadurch gekennzeichnet, dass das gelenkpfannenartige Element aus Hochdruckpolyäthylen ist und der Oberschenkelkopf mit einer Kalotte (20) bedeckt ist, die entweder aus Metall oder aus Kunststoff besteht, unter der Bedingung, dass dieser Kunststoff reibungsmässig mit dem Hochdruckpolyäthylen der Gelenkpfannenhöhlung verträglich ist.

7. Prothese nach einem der vorhergehenden Ansprüche, dadurch gekennzeichnet, dass der obere Teil des gelenkpfannenartigen Elements (1) Formveränderungen in Form von Ausbuchtungen oder Dickenveränderungen erfährt, die zum Verdünnen des Bodens oder Daches des genannten Elements bestimmt sind, damit die Hörner des Elements der physiologischen Bewegung des entsprechenden vorderen und hinteren Horns des Beckens folgen können.

8. Prothese nach einem der vorhergehenden Ansprüche, dadurch gekennzeichnet, dass das gelenkpfannenartige Element aus einem Material besteht, dessen Modul von Young in der Nähe des Moduls des Knochens liegt.

Fig. 1

Fig. 2

Fig. 3

Fig. 4

Fig. 5

Fig. 6

Fig. 7

Fig. 8